(19) Europäisches Patentamt
European Patent Office
Office européen des brevets

(11) **EP 4 729 481 A1**

(12) **EUROPEAN PATENT APPLICATION**
published in accordance with Art. 153(4) EPC

(43) Date of publication:
**22.04.2026 Bulletin 2026/17**

(21) Application number: **23941427.9**

(22) Date of filing: **28.12.2023**

(51) International Patent Classification (IPC):
***C01B 39/10*** *(2006.01)* ***C07C 2/86*** *(2006.01)*

(52) Cooperative Patent Classification (CPC):
**B01J 29/70; B01J 29/78; C01B 39/02; C01B 39/10; C01B 39/46; C07C 1/247; C07C 2/86; C07C 15/08; C07D 493/08**

(86) International application number:
**PCT/CN2023/142795**

(87) International publication number:
**WO 2024/255207 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.06.2023 CN 202310715985**

(71) Applicants:
- **CHINA PETROLEUM & CHEMICAL CORPORATION**
  **Chaoyang District**
  **Beijing 100728 (CN)**
- **Sinopec (Shangai) Research Institute of Petrochemical Technology Co., Ltd.**
  **Shanghai 201208 (CN)**

(72) Inventors:
- **YANG, Weimin**
  **Shanghai 201208 (CN)**
- **FENG, Xinqiang**
  **Shanghai 201208 (CN)**
- **LI, Xiangcheng**
  **Shanghai 201208 (CN)**
- **WANG, Zhendong**
  **Shanghai 201208 (CN)**
- **LIU, Chuang**
  **Shanghai 201208 (CN)**
- **XU, Rui**
  **Shanghai 201208 (CN)**
- **HAN, Xiao**
  **Shanghai 201208 (CN)**

(74) Representative: **Hoffmann Eitle**
**Patent- und Rechtsanwälte PartmbB**
**Arabellastraße 30**
**81925 München (DE)**

# (54) P-MODIFIED MWW MOLECULAR SIEVE, AND PREPARATION METHOD THEREFOR AND USE THEREOF

(57) The present invention relates to the field of molecular sieves, and particularly relates to a P-modified MWW molecular sieve, and a preparation method therefor and the use thereof. The molecular sieve shows $A_{P\delta\text{-}7}/A_P \geq 40\%$, and preferably $\geq 50\%$, wherein $A_{P\delta\text{-}7}$ represents a peak area of a P-species signal peak, which has a chemical shift within the range of -22 ppm to 8 ppm in a $^{31}P$ MAS NMR spectrogram, and $A_P$ represents the total peak area of all signal peaks in the $^{31}P$ MAS NMR spectrogram. A catalyst which contains the molecular sieve of the present invention has high levels of activity, selectivity and stability, and has an excellent carbon deposition resistance capability.

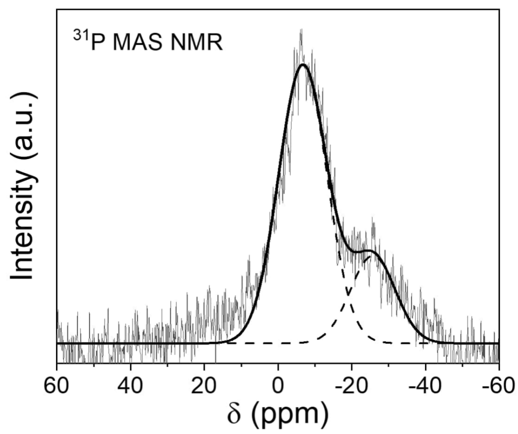


Fig. 3



Processed by Luminess, 75001 PARIS (FR)

## Description

### Technical Field

The invention relates to the field of molecular sieves, and in particular to a P-modified MWW molecular sieve, a preparation method therefor and use thereof, in particular application in the bio-based synthesis of p-xylene.

### Background Art

Since the beginning of the 21st century, countries have been actively seeking new technologies that use biomass, a renewable resource, as raw materials. In the chemical industry, the technologies of replacing traditional petroleum-based chemicals with bio-based chemicals has flourished. Paraxylene (PX) is one of the most important bulk chemicals. It is mainly used to produce terephthalic acid via oxidation, and the latter is further polymerized with ethylene glycol to produce polyethylene terephthalate polyester, which is widely used in the polyester fiber and polyester plastic industries. China's demand for PX in 2021 is about 35 million tons. At present, most of PX comes from petroleum raw materials, but bio-based PX is beginning to be used in the production of renewable plastic bottles, polyester fibers, etc. A promising route is to prepare PX from biomass-derived 2,5-dimethylfuran (DMF) and ethylene.

DMF and ethylene first undergo a Diels-Alder cycloaddition reaction to generate a cycloaddition intermediate, oxa-norbornene, which then undergoes a dehydration reaction under acid catalysis to generate PX and water. Since 2,5-hexadione and DMF can be converted under acidic conditions into each other, 2,5-hexadione can also be used as a raw material to react with ethylene under solid acid catalysis to prepare PX. In a reaction system where DMF and ethylene react with each other to generate PX, side reactions include (1) hydrolysis of DMF to 2,5-hexadione and further dehydration to 3-methyl-2-cyclopentene-1-one; (2) alkylation side reactions, i.e., alkylation reactions of the cycloaddition intermediate with electron-rich molecules such as ethylene to generate over-alkylation products of PX; (3) oligomerization side reactions, i.e., oligomerizations of DMF or 2,5-hexadione itself; and (4) isomerization of the cycloaddition intermediate. DMF molecules are relatively active, and strong acids prone to cause self-polymerization of DMF molecules and trigger hydrolysis and condensation side reactions of DMF. Appropriate acidities can meet the needs of the main reactions, namely D-A addition and subsequent dehydration, so that the target product PX can be obtained with high selectivity. Therefore, the catalytic material should avoid strong acid sites in design, or modification methods can be used to eliminate strong acid sites on the catalyst. In addition, the oligomerization side reactions can cause serious carbon deposition problems, and the catalytic material will be rapidly deactivated due to carbon deposition on the surface. So, the catalyst is required to have a large external specific surface area to resist carbon deposition.

Traditional silicon-aluminum molecular sieves usually have a small external specific surface area and a strong acidity, while SCM-1 is a two-dimensional layered material with a huge external specific surface area, but its disadvantage is that it has a strong acidity. Although CN113831238A and CN113831308A successfully prepare modified SCM-1 materials, Sn-Al-SCM-1 and Zr-Al-SCM-1, strong acid sites still exist, and the problem of eliminating strong acid sites is not solved. Such catalysts will be difficult to catalyze the reaction of DMF and ethylene to prepare PX with high selectivity.

CN103814005A discloses a method for producing paraxylene, toluene and other compounds from renewable resources and ethylene by using homogeneous metal salts as Lewis acid catalysts. CN102482177B discloses the preparation of PX from DMF and ethylene by using acid-washed activated carbon as a catalyst. WO2014/043468A1 discloses the preparation of PX from DMF and ethylene by using supported or unsupported metal salt catalysts. CN109569677B discloses the use of phosphate-type solid acid catalysts in the reaction of DMF and ethylene to prepare PX.

Some other documents related to the background art include CN107626341A, CN115385771A, CN102233274A, US20140194276A1 and CN109678172A.

However, there is still a need for a solid catalyst suitable for catalyzing the reaction of DMF and ethylene to produce PX, which catalyst exhibits desirable properties such as high activity, selectivity and stability and good resistance to carbon deposition.

### Summary of the Invention

In order to overcome the problems existing in the prior art, such as the excessively high acid strength of molecular sieve catalysts in the bio-based PX synthesis leading to low product selectivity, and the easy loss of catalyst active sites leading to poor stability, the inventors have conducted diligently research. As a result, it has been found that a new class of P-modified MWW molecular sieves is particularly suitable for catalyzing the reaction of 2,5-dimethylfuran and/or 2,5-hexadione with ethylene to prepare p-xylene. Said molecular sieve catalysts have high product selectivity, strong carbon deposition resistance and high cycle stability. Thus, the present invention has been made.

A first aspect of the present invention provides a P-modified MWW molecular sieve, which shows an $A_{P\delta\text{-}7}/A_P$

ratio of larger than or equal to 40%, and preferably of larger than or equal to 50%.

A second aspect of the present invention provides a method for preparing the molecular sieve, comprising: performing P-modification on a H-type MWW molecular sieve as a precursor, wherein prior to the P-modification, part or all of Al is optionally removed and then a non-aluminum metal M is optionally grafted.

A third aspect of the present invention provides use of a catalyst comprising the P-modified MWW molecular sieve in the preparation of paraxylene using 2,5-dimethylfuran and/or 2,5-hexadione as raw materials.

A fourth aspect of the present invention provides a method for bio-based synthesis of paraxylene, comprising reacting 2,5-dimethylfuran and/or 2,5-hexadione with ethylene in the presence of a catalyst comprising the P-modified MWW molecular sieve to provide an effluent comprising paraxylene; and isolating the paraxylene.

The catalyst comprising the molecular sieve of the present invention shows high activity, selectivity and stability, and has excellent resistance to carbon deposition. For example, by using the catalyst of the present invention, 2,5-dimethylfuran and/or 2,5-hexadione can react with ethylene, thereby being efficiently and high-selectively converted into para-xylene (PX). It is believed that the two-dimensional layered structure of the molecular sieve gives the catalyst comprising the molecular sieve good mass transfer performance, which is conducive to the smooth progress of the reaction and has strong resistance to carbon deposition. Compared to a non-P-modified molecular sieve, the P-modified molecular sieve of the present invention has a greatly reduced acid strength, and its appropriate acid strength can effectively reduce the occurrence of strong acid-catalyzed alkylation, isomerization and various polymerization side reactions so that the content of key impurities (such as polyalkylbenzenes, isomerization products of cycloaddition intermediates, oligomers of 2,5-dimethylfuran) is extremely low, not only achieving a high PX selectivity, but also greatly reducing the duty of subsequent separation and purification of PX. The P-modification not only forms new acidic sites with appropriate acid strength and provides a small amount of Bronsted acid, but also the binding of P species and metal plays a role in stabilizing the active sites of the catalyst (no obvious change in activity was observed when the catalyst was recycled).

Brief Description of the Drawings

Fig. 1 is an XRD pattern of the P-Al-SCM-1 molecular sieve obtained in Example 1.
Fig. 2 is a SEM image of the P-Al-SCM-1 molecular sieve obtained in Example 1.
Fig. 3 is a $^{31}P$ MAS NMR spectrum of the P-Al-SCM-1 molecular sieve obtained in Example 1.
Fig. 4 is a $NH_3$-TPD diagram of the P-Al-SCM-1 molecular sieve obtained in Example 1.
Fig. 5 is a Py-FTIR spectrum of the P-Al-SCM-1 molecular sieve obtained in Example 1.
Fig. 6 is a $^{31}P$ MAS NMR spectrum of the P-Al-Beta molecular sieve obtained in Comparative Example 1.

Description of the Preferred Embodiments

The endpoints of ranges and any values disclosed herein are not limited to the precise ranges or values, and these ranges or values should be understood as including values close to these ranges or values. For numerical ranges, the end values of individual ranges, the end values of individual ranges and the individual point values therebetween, and the individual point values can be combined with each other to obtain one or more new numerical ranges, which should be considered as being specifically disclosed herein.

In the first aspect, the present invention provides a P-modified MWW molecular sieve, which shows an $A_{P\delta\text{-}7}/A_P$ ratio of larger than or equal to 40%, and preferably of larger than or equal to 50%, wherein $A_{P\delta\text{-}7}$ represents a peak area of P signal peaks having a chemical shift in the range of -22 ppm to 8 ppm in a $^{31}P$ MAS NMR spectrogram, and $A_P$ represents the total peak area of all signal peaks in the $^{31}P$ MAS NMR spectrogram. It is believed that the signal peaks in the range of -22 ppm to 8 ppm are attributable to those P species connected to a single or two metal atoms but not to another P, i.e., (M-O)$PO(OH)_2$ and $(M\text{-}O)_2$-PO(OH).

In preferred embodiments, the molecular sieve shows an $A_{P\delta\text{-}7}/A_P$ ratio of larger than or equal to 70%. For example, the $A_{P\delta\text{-}7}/A_P$ ratio of the molecular sieve may be 70%, 80%, 90% or 100%.

In the $^{31}P$ MAS NMR spectrogram, the molecular sieve of the present invention also exhibits signal peaks with a chemical shift in the range of -41 ppm to -11 ppm, which are P species signal peaks of pyrophosphate and polyphosphate.

In preferred embodiments, the molecular sieve comprises at least one doping metal selected from the group consisting of Group IVB, Group VB, Group VIB, Group IIIA, and Group IVA metals, preferably Al, Sn, Ti, W, Zr, Nb, Ta, and Ga, further preferably Al, Sn, W, and Nb, and more preferably Sn.

In preferred embodiments, the MWW molecular sieve is at least one selected from the group consisting of MCM-type molecular sieves (e.g., MCM-22 disclosed in CN106517232B, the disclosure of which is incorporated herein by reference), ITQ-type molecular sieves (e.g., ITQ-2 disclosed in WO1997017290A1, the disclosure of which is incorporated herein by reference), and SCM-type molecular sieves (e.g., SCM-1 disclosed in CN104511271B, the disclosure of which is incorporated herein by reference), and preferably SCM-type molecular sieves.

In preferred embodiments, the SCM-type molecular sieve is SCM-1 molecular sieve and/or SCM-2 molecular sieve, and preferably SCM-1.

In preferred embodiments, crystals of the molecular sieve are of two-dimensional layered structure. The two-dimensional layered structure refers to the thin nanosheet structure of the MWW molecular sieves. This layered structure provides a large external specific surface area, which is conducive to mass transfer.

In preferred embodiments, the molecular sieve has an internal specific surface area of from 80 to 300 $m^2/g$, and preferably from 100 to 200 $m^2/g$.

In preferred embodiments, the molecular sieve has an external specific surface area of from 50 to 200 $m^2/g$, and preferably from 100 to 200 $m^2/g$.

In preferred embodiments, the molecular sieve has a molar ratio of Lewis acid/Brønsted acid of at least 1.

In preferred embodiments, the molecular sieve has a total acid amount of from 400 to 900 μmol/g, and preferably from 500 to 700 μmol/g, wherein a strong acid content is $\leq$ 15 mol%, and preferably $\leq$ 10 mol%, based on the total acid amount.

In preferred embodiments, the molecular sieve has a Si/metal molar ratio of from 13 to 100, and preferably from 15 to 50.

In preferred embodiments, the molecular sieve has a Si/P molar ratio of from 15 to 100, and preferably from 20 to 50.

In the second aspect, the present invention provides a method for preparing the molecular sieve of the present invention, comprising: performing P-modification on a H-type MWW molecular sieve as a precursor, wherein prior to the P-modification, part or all of Al is optionally removed and then a non-aluminum metal is optionally grafted.

In preferred embodiments, the H-MWW molecular sieve is prepared by a conventional method in the art, comprising calcining a MWW molecular sieve (e.g., the SCM-1 molecular sieve synthesized by the preparation method described in CN104511271B) to remove the structure directing agent present therein, for example, calcining in air or oxygen at 400 to 700 °C for 1 to 6 h, followed by ion exchange in an ammonium salt solution and then calcination again, wherein conditions for the ion exchange and the re-calcination can be those conventionally selected in the art, for example, exchanging in a 1 mol/L $NH_4Cl$ solution at 80 °C for 1 h, repeating 3 times, and then re-calcining in air or oxygen at 400 to 700 °C for 1 to 6 h, to obtain the H-MWW.

In the present invention, there is no particular limitation on the method for removing part or all of Al. In preferred embodiments, the method for removing part or all of Al comprises first subjecting the H-type MWW molecular sieve to a steam hydrothermal treatment, and then to an acid solution washing.

In the present invention, the acid solution has a wide range of options. Preferably, the acid solution is at least one selected from the group consisting of hydrochloric acid solution, oxalic acid solution, nitric acid solution, sulfuric acid solution and phosphoric acid solution, and preferably nitric acid solution.

In the present invention, conditions for the steam hydrothermal treatment can be those conventionally selected in the art. Preferably, the conditions for the steam hydrothermal treatment include an inert atmosphere. The inert atmosphere refers to a gas that does not participate in the reaction, and in the present invention, it can be nitrogen, air, argon, etc., for example. The role of the gas is to carry the steam through the material.

In preferred embodiments, the conditions for the steam hydrothermal treatment include a steam concentration in gas phase of from 10 to 100 mol%, and preferably from 30 to 70 mol%.

In preferred embodiments, the conditions for the steam hydrothermal treatment include a temperature of from 600 to 900 °C, and preferably from 700 to 800 °C.

In preferred embodiments, the conditions for the steam hydrothermal treatment include a period of time of from 1 to 48 h, and preferably from 6 to 12 h.

In the present invention, conditions for the acid solution washing can be those conventionally selected in the art. Preferably, the conditions for the acid solution washing include a concentration of the acid solution of from 1 mol/L to 14 mol/L.

In preferred embodiments, the washing conditions include a temperature of from 20 to 120 °C, and preferably from 80 to 110 °C.

In preferred embodiments, the washing conditions include a period of time of from 6 to 48 h, and preferably from 12 to 24 h.

In preferred embodiments, the washing conditions include a final Si/Al molar ratio at the end of the washing of from 25 to 180, and preferably from 100 to 180.

In the present invention, the method of grafting the non-aluminum metal M can be a conventional choice in the art. In preferred embodiments, the method of grafting the non-aluminum metal M is a solvent reflux method, comprising placing a molecular sieve, having been subjected to a partially or completely Al-removing, in an organic solution containing a non-aluminum metal M source and then refluxing, followed by a first solid-liquid separation, a first washing, a first drying, and a

first calcination.

In preferred embodiments, the non-aluminum metal M source is a compound of at least one metal selected from the group consisting of Sn, Ti, W, Zr, Nb, Ta and Ga, preferably a compound of Sn and/or W and/or Nb, and more preferably at least one selected from tin tetrachloride, dimethyltin dichloride, niobium pentachloride, and tungsten hexachloride.

In preferred embodiments, the organic solvent is at least one selected from the group consisting of alkanes, aromatic hydrocarbons, and halogenated hydrocarbons, for example, dichloromethane or n-hexane.

In preferred embodiments, the grafted molecular sieve has a Si/M molar ratio of from 13 to 100, and preferably from 15 to 50.

In preferred embodiments, conditions for the first calcination include a temperature of from 400 to 700 °C, and preferably from 500 to 600 °C.

In preferred embodiments, conditions for the first calcination include a period of time of from 3 to 12 h, and preferably from 4 to 8 h.

In the present invention, there is no particular limitation on conditions of the first solid-liquid separation, the first washing, and the first drying, as long as the purpose of the present invention can be achieved.

In the present invention, the P-modification method can be a conventional choice in the art, such as impregnation. Alternatively, in preferred embodiments, the P-modification method comprises placing a MWW molecular sieve, having been subjected optionally to a partially or completely Al-removing treatment and then optionally to a non-aluminum metal M-grafting treatment, in an organic solution containing a phosphorus source and then refluxing, followed by a second solid-liquid separation, a second washing, and a second calcination.

In preferred embodiments, the phosphorus source is at least one selected from the group consisting of dimethylphosphonic acid, phosphorus oxychloride, methylphosphonic acid, trimethylphosphine oxide, trimethylphosphine and phosphorus pentachloride.

In the present invention, there is no particular limitation on the amount of the phosphorus source added, as long as the purpose of the present invention can be achieved. In preferred embodiments, the amount of the phosphorus source added is from 0.05 to 1 mol, preferably from 0.06 to 0.50 mol, and more preferably from 0.07 to 0.30 mol, relative to 1 mol of silicon in the molecular sieve.

In preferred embodiments, the organic solvent of the organic solution is at least one selected from alkanes, aromatic hydrocarbons, and halogenated hydrocarbons. Examples of the organic solvent are dichloromethane and n-hexane.

In the present invention, there is no particular limitation on the reflux time of the P-modification. The reflux time can be, for example, 0.5 to 12 hours.

In preferred embodiments, after the P-modification, the molecular sieve has a Si/P molar ratio of from 15 to 100, and preferably from 20 to 50.

In preferred embodiments, conditions for the second calcination include a temperature of from 400 to 700 °C, and preferably from 500 to 600 °C.

In preferred embodiments, conditions for the second calcination include a period of time of from 3 to 12 hours, and preferably from 4 to 8 hours.

In the present invention, there is no particular limitation on conditions for the second solid-liquid separation and the second washing, as long as the purpose of the present invention can be achieved.

Without being bound by a specific theory, it is believed that the microstructure (two-dimensional layered structure, thickness), pore structure and acidity of the MWW molecular sieve make it particularly suitable to be modified by the P-modification method of the present invention, to provide a molecular sieve catalyst particularly suitable for the bio-based synthesis of PX.

In preferred embodiments, the H-type MWW molecular sieve useful in the present invention is at least one of an H-type MCM molecular sieve, an H-type ITQ molecular sieve and an H-type SCM molecular sieve; preferably an H-type SCM molecular sieve; further preferably an H-type SCM-1 molecular sieve and/or an H-type SCM-2 molecular sieve, and more preferably an H-type SCM-1 molecular sieve.

In the third aspect, the present invention provides use of a catalyst comprising the molecular sieve of the present invention in the preparation of p-xylene by using 2,5-dimethylfuran and/or 2,5-hexadione as raw materials.

Reaction Scheme:

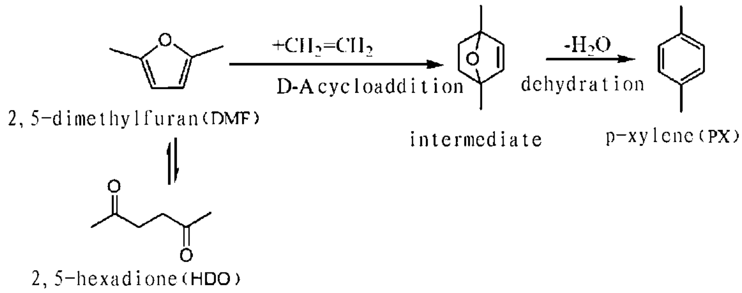


In the fourth aspect, the present invention provides a method for bio-based synthesis of paraxylene, comprising reacting 2,5-dimethylfuran and/or 2,5-hexadione with ethylene in the presence of a catalyst comprising the P-modified MWW molecular sieve of the present invention to provide an effluent comprising paraxylene; and isolating the paraxylene.

The compounding of the P-modified MWW molecular sieve of the present invention into a catalyst suitable for use in an industrial process can be done according to techniques known in the art. For example, the molecular sieve of the present invention can be mixed with an appropriate amount of a binder, molded, dried and calcined to obtain the catalyst. The binder can be at least one of alumina, silica, titania and a precursor thereof. The mass of the binder can account for 10 to 60%, for example 10 to 40%, of the mass of the molecular sieve. In order to facilitate the molding, molding aids such as cellulose, water, etc. can also be introduced.

The drying and calcining in the catalyst preparation can be carried out in a conventional manner. Preferably, drying conditions include a drying temperature of 90 to 160 °C and a drying time of 6 to 15 hours, and calcining conditions include an oxygen-containing atmosphere such as air, a calcination temperature of 300 to 550 °C and a calcination time of 2 to 5 hours.

The procedure and reaction conditions for reacting 2,5-dimethylfuran and/or 2,5-hexadione with ethylene in the presence of a molecular sieve catalyst to provide an effluent comprising p-xylene as well as the procedure and conditions for separating p-xylene from the reaction effluent are known in the art.

In the present disclosure including the following examples, the following analysis and testing methods are used.

The components of a liquid reaction mixture are analyzed qualitatively by using Agilent 7890A gas chromatography-mass spectrometry instrument (GC-MS) of Agilent, USA, and the chromatographic column is a Wax polar capillary column. Quantitative analysis is performed on Agilent 7890B gas chromatograph (GC) of Agilent, USA, equipped with a hydrogen ion flame detector (FID) and a Wax polar capillary column. Standard substances are used to plot calibration curves, and molar amounts of the components of samples are then measured. The conversion rate and selectivity of the substances are calculated based on the molar amounts of the substances measured.

The conversion rate of raw material is calculated according to the following equation:

Raw material conversion rate % = (molar amount of initially added raw material - molar amount of unconverted raw material) / (molar amount of initially added raw material) $\times$ 100%.

The product selectivity is calculated according to the following equation:

Product selectivity % = (molar amount of product generated by the reaction)/(molar amount of initially added raw material - molar amount of unconverted raw material) $\times$ 100%.

As used herein, the term "raw material" refers to 2,5-dimethylfuran and/or 2,5-hexadione.

XRD patterns are acquired on a D8 Advance X-ray diffractometer available from Bruker, German, using CuK$\alpha$ radiation source ($\lambda$ = 1.54 Å).

The composition of the molecular sieve is measured on an Agilent 725-ES inductively coupled plasma atomic emission spectrometer (ICP). The molecular sieve sample is resolved with hydrofluoric acid and the element contents are expressed on molar basis.

Scanning electron microscopy (SEM) images are acquired on a S-4800II field emission scanning electron microscope.

Solid state $^{31}P$ magic angle spinning nuclear magnetic resonance ($^{31}P$ MAS NMR) spectra are acquired on a Varian 400 MHz NMR instrument, and $H_3PO_4$ is used as a reference for the chemical shift of $^{31}P$.

The $NH_3$ programmed temperature desorption ($NH_3$-TPD) experiment is carried out on a TPD/TPR Altamira AMI-3300 instrument, and a total acid amount is determined through calculation based on the obtained diagram.

The pyridine adsorption infrared (py-FTIR) method is used to determine the acid species of a catalyst, and the py-FTIR spectrum is acquired on a Nicolet Model 710 spectrometer. The specific operation steps are as follows: a) Sample preparation and pretreatment: about 15 mg of sample is pressed into a thin disc with a diameter of 13 mm and loaded into the infrared sample tank; then the sample is pretreated at 400 °C in a vacuum cell for 2 hours; the sample is scanned at 100 °C, 200 °C, and 400 °C to acquire background spectra. b) After the sample tank is cooled to room temperature, pyridine is adsorbed for 10 minutes; vacuum desorption is performed at 100°C for 10 minutes, and an infrared absorption spectrum is scanned and recorded; then infrared absorption spectra are scanned and recorded successively at 200 °C and 400 °C. The differential spectra before and after pyridine adsorption are the acquired Py-FTIR spectra. The peak at 1450 $cm^{-1}$ is the absorption peak of L acid, and the peak at 1540 $cm^{-1}$ is the absorption peak of B acid. The ratio of the total absorption peak area at 1450 $cm^{-1}$ and 1540 $cm^{-1}$ of pyridine that has not been desorbed at 400 °C to the total absorption peak area of pyridine at 100 °C is the strong acid proportion, and the ratio of the L acid absorption peak area to the B acid absorption peak area is recorded as the L/B ratio.

The specific surface area and external specific surface area of the molecular sieve are measured by nitrogen physical adsorption and desorption method: the adsorption and desorption curve is measured on Micromeretic ASAP 2020M physical adsorption instrument, the total specific surface area is obtained by BET equation, the external specific surface area is calculated by t-plot equation, and the difference between the two is the internal specific surface area. Prior to the measurement, the sample is pretreated at 350 °C in vacuum for 6 hours.

Examples

The present invention will be described in detail below by way of examples, but the scope of the present invention is not limited thereto.

Example 1

According to the preparation method described in patent CN 104511271B, a SCM-1 molecular sieve raw powder was synthesized and then calcined to remove structure directing agent, and then ion exchange and calcination were performed to obtain H-SCM-1. It was found by ICP measurement that the molecular sieve had a silicon/aluminum ratio (atomic ratio) of 15.2.

A P-modified molecular sieve, P-Al-SCM-1, was prepared from the H-SCM-1 by the following steps:

(1) High-temperature steam hydrothermal treatment of H-SCM-1 molecular sieve: treating at 700 °C under nitrogen atmosphere and 60% water vapor partial pressure for 10 h.

(2) Preparation of P-Al-SCM-1: The above hydrothermally treated molecular sieve was directly refluxed in a dichloromethane solution containing a P-source, that is, a dimethylphosphonic acid solution was added for treatment for 1 hour, the amount of the P-source added being 1/10 of the molar amount of silicon in the molecular sieve, and the content of the molecular sieve in the solution being 1 g of molecular sieve per 50 mL of solution. Centrifugation and washing with dichloromethane gave a solid product, which was calcined at 550 °C for 4 hours to obtain P-Al-SCM-1. ICP measurement revealed a Si/Al ratio of 15.2 and a Si/P ratio of 21.2. An XRD pattern is shown in Figure 1, showing that the MWW topological structure is still well maintained. A SEM image (see Figure 2) shows that the obtained molecular sieve has a two-dimensional layered structure, and BET measurement revealed an internal specific surface area of 93 $m^2$/g and an external specific surface area of 136 $m^2$/g. As shown in Figure 3, a $^{31}$P MAS NMR spectrum of the catalyst has an $A_{P\delta-7}/A_P$ of 77%. A $NH_3$-TPD curve is shown in Figure 4, and the total acid amount is calculated to be 684 $\mu$mol/g. Furthermore, it can be seen that less $NH_3$ is adsorbed above 300 °C, indicating that there are fewer strong acid sites. A pyridine-IR spectrum (see Figure 5) showed that the overall L/B ratio was 1.9 at 100°C. What are still not desorbed at 400°C are pyridine molecules adsorbed at strong acid sites, and thus it was calculated that strong acid sites accounted for 9 %.

Example 2

A P-Al-SCM-1 molecular sieve was prepared by the procedure described in Example 1, except that the H-SCM-1 molecular sieve was treated at 600 °C under nitrogen atmosphere and 50 % water vapor partial pressure for 48h. ICP measurement revealed a Si/Al ratio of 15.2 and a Si/P ratio of 20.5. BET measurement revealed an internal specific surface area of 105 $m^2$/g and an external specific surface area of 127 $m^2$/g. It was found that the catalyst had an $A_{P\delta}$-7/$A_P$ of 72%. A total acid amount calculated from a $NH_3$-TPD curve was 674 $\mu$mol/g. A pyridine-IR spectrum showed that the L/B ratio was 2.4, and it was calculated that strong acid sites accounteds for 12 %.

Example 3

A P-Al-SCM-1 molecular sieve was prepared by the procedure described in Example 1, except that the H-SCM-1 molecular sieve was refluxed in an n-hexane solution containing phosphorus oxychloride for 8 hours. ICP measurement revealed a Si/Al ratio of 15.2 and a Si/P ratio of 18.7. BET measurement revealed an internal specific surface area of 84 $m^2/g$ and an external specific surface area of 108 $m^2/g$. It was found that the catalyst had an $A_{P\delta\text{-}7}/A_P$ of 79%. A total acid amount calculated from a $NH_3$-TPD curve was 754 $\mu$mol/g. A pyridine-IR spectrum showed that the L/B ratio was 1.8, and it was calculated that strong acid sites accounted for 7 %.

Example 4

According to the preparation method described in patent CN 104511271B, a SCM-1 molecular sieve raw powder was synthesized and then calcined to remove structure directing agent, and then ion exchange and calcination were performed to obtain H-SCM-1. It was found by ICP measurement that the molecular sieve had a silicon/aluminum ratio (atomic ratio) of 15.2.

A P-modified molecular sieve, P-Sn-SCM-1, was prepared from the H-SCM-1 by the following steps:

(1) High-temperature steam hydrothermal treatment of H-SCM-1 molecular sieve: treating at 900 °C under nitrogen atmosphere and 10% water vapor partial pressure for 10 h.

(2) Partial removal of Al with acid solution: washing the hydrothermally treated H-SCM-1 molecular sieve with 14 M concentrated nitric acid at 120 °C for 48 h, the amount of the molecular sieve in the acid solution being 1 g of molecular sieve per 30 mL of acid solution. The final Si/Al ratio was measured to be 142.

(3) Preparation of Sn-SCM-1 by solvent reflux method: the above dealuminated SCM-1 molecular sieve was refluxed in a solution of anhydrous $SnCl_4$ in dichloromethane, the amount of the molecular sieve in the solution being 1g of molecular sieve per 50 mL of solution. Then, it was washed with dichloromethane, dried, and calcined at 550 °C for 4 h to obtain a Sn-SCM-1 molecular sieve.

(4) Under the condition of reflux in dichloromethane solution, the Sn-SCM-1 molecular sieve was treated with dimethyl phosphonic acid for 1 hour, the amount of the dimethylphosphonic acid added being 1/10 of the molar amount of silicon in the molecular sieve. Centrifugation and washing with dichloromethane gave a solid product, which was calcined at 550 °C for 4 hours to obtain the P-Al-SCM-1. ICP measurement revealed a Si/Sn ratio of 35.3 and a Si/P ratio of 21.6. BET measurement revealed an internal specific surface area of 101 $m^2/g$ and an external specific surface area of 116 $m^2/g$. It was found that the catalyst had an $A_{P\delta\text{-}7}/A_P$ of 85%. A total acid amount calculated from a $NH_3$-TPD curve was 641 $\mu$mol/g. A pyridine-IR spectrum showed that the L/B ratio was 2.8, and it was calculated that strong acid sites accounted for 6 %.

Example 5

A P-Sn-SCM-1 was prepared according to the procedure described in Example 4, except that the partial removal of Al with acid solution was carried out by using 1M nitric acid at 80 °C for 12h and the final Si/Al ratio was 25.5. ICP measurement revealed a Si/Sn ratio of 47.1 and a Si/P ratio of 28.9. BET measurement revealed an internal specific surface area of 124 $m^2/g$ and an external specific surface area of 178 $m^2/g$. It was found that the catalyst had an $A_{P\delta\text{-}7}/A_P$ of 70%. A total acid amount calculated from a $NH_3$-TPD curve was 852 $\mu$mol/g. A pyridine-IR spectrum showed that the L/B ratio was 2.2, and it was calculated that strong acid sites accounted for 11%.

Example 6

A P-Sn-SCM-1 was prepared according to the procedure described in Example 4, except that dimethyltin dichloride refluxing treatment was used when preparing Sn-SCM-1. ICP measurement revealed a Si/Sn ratio of 42.6 and a Si/P ratio of 35.6. BET measurement revealed an internal specific surface area of 135 $m^2/g$ and an external specific surface area of 168 $m^2/g$. It was found that the catalyst had an $A_{P\delta\text{-}7}/A_P$ of 75%. A total acid amount calculated from a $NH_3$-TPD curve was 679 $\mu$mol/g. A pyridine-IR spectrum showed that the L/B ratio was 3.1, and it was calculated that strong acid sites accounted for 9%.

Example 7

A P-Ti-SCM-1 was prepared according to the procedure described in Example 4, except that a Ti-SCM-1 intermediate was prepared by refluxing in a solution of $TiCl_4$ in dichloromethane. ICP measurement revealed a Si/Ti ratio of 27.8 and a Si/P ratio of 23.6. BET measurement revealed an internal specific surface area of 145 $m^2/g$ and an external

specific surface area of 179 $m^2$/g. It was found that the catalyst had an $A_{P\delta\text{-}7}/A_P$ of 76%. A total acid amount calculated from a $NH_3$-TPD curve was 705 μmol/g. A pyridine-IR spectrum showed that the L/B ratio was 1.9, and it was calculated that strong acid sites accounted for 8%.

Example 8

A P-Nb-SCM-1 was prepared according to the procedure described in Example 4, except that a Nb-SCM-1 intermediate was prepared by refluxing in a solution of $NbCl_5$ in dichloromethane. ICP measurement revealed a Si/Nb ratio of 29.0 and a Si/P ratio of 25.8. BET measurement revealed an internal specific surface area of 94 $m^2$/g and an external specific surface area of 141 $m^2$/g. It was found that the catalyst had an $A_{P\delta\text{-}7}/A_P$ of 74%. A total acid amount calculated from a $NH_3$-TPD curve was 478 μmol/g. A pyridine-IR spectrum showed that the L/B ratio was 2.1, and it was calculated that strong acid sites accounted for 11%.

Example 9

A P-W-SCM-1 was prepared according to the procedure described in Example 4, except that a W-SCM-1 intermediate was prepared by refluxing in a solution of $WCl_6$ in dichloromethane. ICP measurement revealed a Si/W ratio of 22.5 and a Si/P ratio of 20.6. BET measurement revealed an internal specific surface area of 85 $m^2$/g and an external specific surface area of 128 $m^2$/g. It was found that the catalyst had an $A_{P\delta\text{-}7}/A_P$ of 78%. A total acid amount calculated from a $NH_3$-TPD curve was 490 μmol/g. A pyridine-IR spectrum showed that the L/B ratio was 3.3, and it was calculated that strong acid sites accounted for 9%.

Example 10

The molecular sieve catalysts prepared above in Example 1-9 were used for the reaction of preparing PX from DMF and ethylene, with reaction conditions including: a reaction solvent of n-heptane, a mass concentration of DMF in the solvent of 15%; a mass ratio of DMF to catalyst of 6:1; a reaction temperature of 250 °C; and a reaction time of 12 h. 0.5 g of the catalyst of Examples 1-9, 3.0 g of DMF and 17 g of n-heptane were added to a magnetically stirred autoclave, and 2.0 MPa of ethylene was charged thereinto. The reaction was carried out at 250 °C for 12 h, and the liquid reaction mixture was analyzed by gas chromatography to determine DMF conversion and PX selectivity. The results are shown in Table 1 below.

Table 1

| Catalyst No. | DMF Conversion (%) | PX Selectivity (%) |
|---|---|---|
| Example 1 | 98.1 | 97.4 |
| Example 2 | 98.3 | 96.9 |
| Example 3 | 97.0 | 97.7 |
| Example 4 | 97.1 | 98.0 |
| Example 5 | 96.9 | 95.4 |
| Example 6 | 98.1 | 97.7 |
| Example 7 | 93.5 | 94.9 |
| Example 8 | 97.6 | 92.8 |
| Example 9 | 96.7 | 94.1 |

Example 11

This example used the P-Al-SCM-1 molecular sieve prepared in Example 1 as a catalyst. 0.5 g of the catalyst, 3.0 g of 2,5-hexadione (HDO) and 17 g of n-heptane were added to a magnetically stirred autoclave, and 2.0 MPa of ethylene was charged thereinto. The reaction was carried out at 250 °C for 12 h, and the liquid reaction mixture was analyzed by gas chromatography, finding an HDO conversion of 97.5% and a PX selectivity of 96.5%.

This example demonstrates that the HDO is also a useful raw material in the preparation of PX.

Example 12

This example used the P-Al-SCM-1 molecular sieve prepared in Example 1 as a catalyst. 2.0 g of the catalyst and 15.0 g of DMF were added to a magnetically stirred autoclave, and 6.0 MPa of ethylene was charged thereinto. The reaction was carried out at 250 °C for 12 h, and the liquid reaction mixture was analyzed by gas chromatography, finding a DMF conversion of 48.2% and a PX selectivity of 92.3%.

This example demonstrates that in the preparation of PX, a higher PX selectivity can be achieved when the raw material is converted in the presence of an organic solvent. However, when no organic solvent is present, a high reactant concentration and a high product concentration can be achieved and a yield per unit reactor volume is higher.

Example 13

This example used the P-Al-SCM-1 molecular sieve prepared in Example 1 as a catalyst. 1.0 g of the catalyst, 3.0 g of HDO and 17 g of n-hexane were added to a magnetically stirred autoclave, and 3.0 MPa of ethylene was charged thereinto. The reaction was carried out at 250 °C for 24 h, and the liquid reaction mixture was analyzed by gas chromatography, finding an HDO conversion of 98.2% and a PX selectivity of 96.7%.

Example 14

This example used the P-Al-SCM-1 molecular sieve prepared in Example 1 as a catalyst. 0.5 g of the catalyst, 3.0 g of DMF and 17.0 g of tetrahydrofuran were added to a magnetically stirred autoclave, and 4.0 MPa of ethylene was charged thereinto. The reaction was carried out at 300 °C for 3 h, and the liquid reaction mixture was analyzed by gas chromatography, finding a DMF conversion of 95.2% and a PX selectivity of 97.0%.

The experimental results of Examples 13 and 14 demonstrate that those commonly used organic solvents are suitable for this reaction. It can be known by increasing reaction temperature to 300 °C in Example 14, increasing the reaction temperature can shorten the reaction time, but the disadvantage is that the reactor should have better high-temperature resistance.

Example 15

This example used the P-Al-SCM-1 molecular sieve prepared in Example 1 as a catalyst. 0.5 g of the catalyst, 3.0 g of HDO and 17 g of tetrahydrofuran were added to a magnetically stirred autoclave, and 1.0 MPa of ethylene was charged thereinto. The reaction was carried out at 270 °C for 24 h, and the liquid reaction mixture was analyzed by gas chromatography, finding an HDO conversion of 98.1% and a PX selectivity of 96.1%.

Example 16

This example used the P-Al-SCM-1 molecular sieve prepared in Example 1 as a catalyst. 0.5 g of the catalyst, 3.0 g of DMF and 17 g of tetrahydrofuran were added to a magnetically stirred autoclave, and 3.0 MPa of ethylene was charged thereinto. The reaction was carried out at 235 °C for 24 h, and the liquid reaction mixture was analyzed by gas chromatography, finding a DMF conversion of 94.3% and a PX selectivity of 96.7%.

Example 17

In this example, cycle activities of the P-Al-SCM-1 molecular sieve catalyst prepared in Example 1 for the reaction of preparing PX from DMF and ethylene were tested. Reaction conditions include: a reaction solvent of n-heptane; a mass concentration of DMF in solvent of 15%; a mass ratio of DMF to catalyst of 6:1; a reaction temperature of 250 °C; and a reaction time of 12 h. 0.5 g of the catalyst, 3.0 g of DMF and 17 g of n-heptane were added to a magnetically stirred autoclave, and 2.0 MPa of ethylene was charged thereinto. The reaction was carried out at 250 °C for 12 h, and the liquid reaction mixture was analyzed by gas chromatography to determine DMF conversion and PX selectivity. After the reaction was completed, the spent catalyst was isolated by centrifugation, calcined to remove carbon deposits thereon, and reused in a next cycle. The results are shown in Table 2 below. It can be seen that the DMF conversion and PX selectivity were basically unchanged for at least 5 cycles, indicating that the catalyst has an excellent recycle stability.

Table 2

| Cycle Number | DMF Conversion (%) | PX Selectivity (%) |
|---|---|---|
| 1 | 98.1 | 97.4 |

(continued)

| Cycle Number | DMF Conversion (%) | PX Selectivity (%) |
|---|---|---|
| 2 | 98.0 | 97.6 |
| 3 | 98.3 | 97.0 |
| 4 | 97.5 | 97.3 |
| 5 | 98.0 | 97.3 |

Example 18

Example 1 was repeated, except that in step (2), the P-modification was carried out by an incipient wetness impregnation method using a solution of dimethylphosphoric acid in dichloromethane, with the amount of the P-source added being 1/10 of the molar amount of silicon in the molecular sieve. After the impregnation, drying and calcination at 550 °C for 4h gave a P-Al-SCM-1. ICP measurement revealed a Si/Al ratio of 15.2 and a Si/P ratio of 10.0. BET measurement revealed an internal specific surface area of 81 $m^2/g$ and an external specific surface area of 102 $m^2/g$. A $^{31}P$ MAS NMR spectrum of the catalyst showed an $A_{P\delta-7}/A_P$ of 43%. A total acid amount calculated from a $NH_3$-TPD curve was 863 μmol/g. A pyridine-IR spectrum showed that the L/B ratio was 1.1, and it was calculated that strong acid sites accounted for 8%.

0.5 g of the catalyst, 3.0 g of DMF and 17 g of n-heptane were added to a magnetically stirred autoclave, and 2.0 MPa of ethylene was charged thereinto. The reaction was carried out at 250 °C for 12 h, and the liquid reaction mixture was analyzed by gas chromatography, finding a DMF conversion of 90.2% and a PX selectivity of 76%.

Comparative Example 1

Example 1 was repeated, except that a Beta molecular sieve available from Tianjin Nanhua Catalyst Co., Ltd. was used to provide a P-modified molecular sieve, P-Al-Beta.

ICP measurement revealed a Si/Al ratio of 18.8 and a Si/P ratio of 24.3. BET measurement revealed an internal specific surface area of 271 $m^2/g$ and an external specific surface area of 19 $m^2/g$. A $^{31}P$ MAS NMR spectrum of the catalyst is shown in Figure 6, which shows an $A_{P\delta-7}/A_P$ of 31%. A total acid amount calculated from a $NH_3$-TPD curve was 598 μmol/g. A pyridine-IR spectrum showed that the L/B ratio was 1.4, and it was calculated that strong acid sites accounted for 19%.

0.5 g of the catalyst, 3.0 g of DMF and 17 g of n-heptane were added to a magnetically stirred autoclave, and 2.0 MPa of ethylene was charged thereinto. The reaction was carried out at 250 °C for 12 h, and the liquid reaction mixture was analyzed by gas chromatography, finding a DMF conversion of 77.1% and a PX selectivity of 68.2%.

It can be seen from the results of the examples that the molecular sieves prepared by the inventive method have relatively large external specific surface areas and appropriate acidities, and in particular have excellent characteristics in catalyzing the reaction of 2,5-dimethylfuran and/or 2,5-hexadione with ethylene to produce p-xylene. Compared to the catalyst of the comparative example, the inventive molecular sieves achieve significantly better effects.

The preferred embodiments of the present invention are described in detail above, but the present invention is not limited thereto. Within the technical concept of the present invention, the technical solutions of the present invention can be subjected to a variety of simple modifications, including the combination of various technical features in any other suitable manner, and these simple modifications and combinations should also be regarded as contents disclosed by the present invention and belong to the protection scope of the present invention.

**Claims**

1. A P-modified MWW molecular sieve, **characterized in that** the molecular sieve shows an $A_{P\delta-7}/A_P$ ratio of larger than or equal to 40%, and preferably of larger than or equal to 50%, wherein $A_{P\delta-7}$ represents a peak area of signal peaks having a chemical shift in the range of -22 ppm to 8 ppm in a $^{31}P$ MAS NMR spectrogram, and $A_P$ represents the total peak area of all signal peaks in the $^{31}P$ MAS NMR spectrogram.

2. The molecular sieve according to claim 1, wherein

   the molecular sieve shows the $A_{P\delta-7}/A_P$ ratio of larger than or equal to 70%; and/or
   the molecular sieve comprises at least one doping metal selected from the group consisting of Group IVB metals,

Group VB metals, Group VIB metals, Group IIIA metals, and Group IVA metals, preferably Al, Sn, Ti, W, Zr, Nb, Ta, Ga, and more preferably Al, Sn, W and Nb; and/or
the MWW molecular sieve is at least one selected from the group consisting of MCM molecular sieves, ITQ molecular sieves, and SCM molecular sieves, preferably SCM molecular sieves, and further preferably, the SCM molecular sieve is selected from SCM-1 molecular sieves and/or SCM-2 molecular sieves, and more preferably SCM-1 molecular sieves.

**3.** The molecular sieve according to claim 1 or 2, wherein

crystals of the molecular sieve are of two-dimensional layered structure; and/or
the molecular sieve has an internal specific surface area of from 80 to 300 $m^2/g$, and preferably from 100 to 200 $m^2/g$; and/or
the molecular sieve has an external specific surface area of from 50 to 200 $m^2/g$, and preferably from 100 to 200 $m^2/g$; and/or
the molecular sieve has a molar ratio of Lewis acid/Brensted acid of at least 1; and/or
the molecular sieve has a total acid amount of from 400 to 900 $\mu$mol/g, and preferably from 500 to 700 $\mu$mol/g, wherein a strong acid content is $\leq$15%, and preferably $\leq$10%, based on the total acid amount.

**4.** The molecular sieve according to any one of claims 1 to 3, wherein

the molecular sieve has a Si/metal molar ratio of from 13 to 100, and preferably from 15 to 50; and/or
the molecular sieve has a Si/P molar ratio of from 15 to 100, and preferably from 20 to 50.

**5.** A method for preparing the molecular sieve according to any one of claims 1 to 4, comprising: performing P-modification on a H-type MWW molecular sieve as a precursor, wherein prior to the P-modification, part or all of Al is optionally removed and then a non-aluminum metal is optionally grafted.

**6.** The method according to claim 5, wherein

a method for removing part or all of Al comprises first subjecting the H-type MWW molecular sieve to a steam hydrothermal treatment, and then to an acid solution washing;
preferably, the acid solution is at least one selected from the group consisting of hydrochloric acid solution, oxalic acid solution, nitric acid solution, sulfuric acid solution and phosphoric acid solution, and preferably nitric acid solution; and/or
conditions for the steam hydrothermal treatment include:

a steam concentration in gas phase of from 10 to 100 mol%, and preferably from 30 to 70 mol%; and/or
a temperature of from 600 to 900 °C, and preferably from 700 to 800 °C; and/or
a period of time of from 1 to 48 h, and preferably from 6 to 12 h; and/or
conditions for the washing include:

a concentration of the acid solution of from 1 mol/L to 14 mol/L; and/or
a temperature of from 20 to 120 °C, and preferably from 80 to 110°C; and/or
a period of time of from 6 to 48h, and preferably from 12 to 24h; and/or
a final Si/Al molar ratio at the end of the washing of from 25 to 180, and preferably from 100 to 180.

**7.** The method according to claim 5 or 6, wherein

a method for grafting the non-aluminum metal M is a solvent reflux method, comprising placing a molecular sieve, having been subjected to a partially or completely Al-removing, in an organic solution containing a non-aluminum metal M source and then refluxing, followed by a first solid-liquid separation, a first washing, a first drying, and a first calcination; preferably,
the non-aluminum metal M source is a compound of at least one metal selected from the group consisting of Sn, Ti, W, Zr, Nb, Ta and Ga; and/or
the organic solvent is at least one selected from the group consisting of alkanes, aromatic hydrocarbons, and halogenated hydrocarbons; and/or
after the grafting, the molecular sieve has a Si/M molar ratio of from 13 to 100, and preferably from 15 to 50; and/or
conditions for the first calcination include:

a temperature of from 400 to 700 °C, and preferably from 500 to 600°C; and/or
a period of time of from 3 to 12 h, and preferably from 4 to 8 h.

**8.** The method according to any one of claims 5 to 7, wherein

a method for the P-modification comprises placing a molecular sieve, having been subjected optionally to a partially or completely Al-removing treatment and then optionally to a non-aluminum metal M-grafting treatment, in an organic solution containing a phosphorus source and then refluxing, followed by a second solid-liquid separation, a second washing, and a second calcination; preferably,
the phosphorus source is at least one selected from the group consisting of dimethylphosphonic acid, phosphorus oxychloride, methylphosphonic acid, trimethylphosphine oxide, trimethylphosphine and phosphorus pentachloride; and/or
an organic solvent is at least one selected from the group consisting of alkanes, aromatic hydrocarbons, and halogenated hydrocarbons; and/or
after the P-modification, the molecular sieve has a Si/P molar ratio of from 15 to 100, and preferably from 20 to 50; and/or
conditions for the second calcination include:

a temperature of from 400 to 700 °C, and preferably from 500 to 600 °C; and/or
a period of time of from 3 to 12 h, and preferably from 4 to 8 h.

**9.** The method according to any one of claims 5 to 8, wherein the H-type MWW molecular sieve is at least one of an H-type MCM molecular sieve, an H-type ITQ molecular sieve and an H-type SCM molecular sieve; preferably an H-type SCM molecular sieve; further preferably an H-type SCM-1 molecular sieve and/or an H-type SCM-2 molecular sieve, and more preferably an H-type SCM-1 molecular sieve.

**10.** A method for bio-based synthesis of paraxylene, comprising reacting 2,5-dimethylfuran and/or 2,5-hexadione with ethylene in the presence of a catalyst comprising the P-modified MWW molecular sieve according to any one of claims 1 to 4 to provide an effluent comprising paraxylene; and isolating the paraxylene.

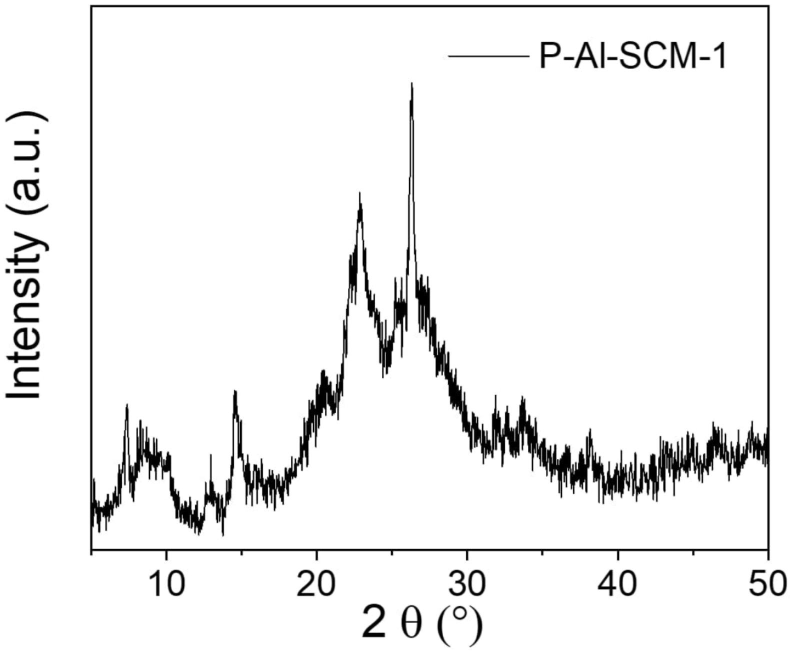


Fig. 1

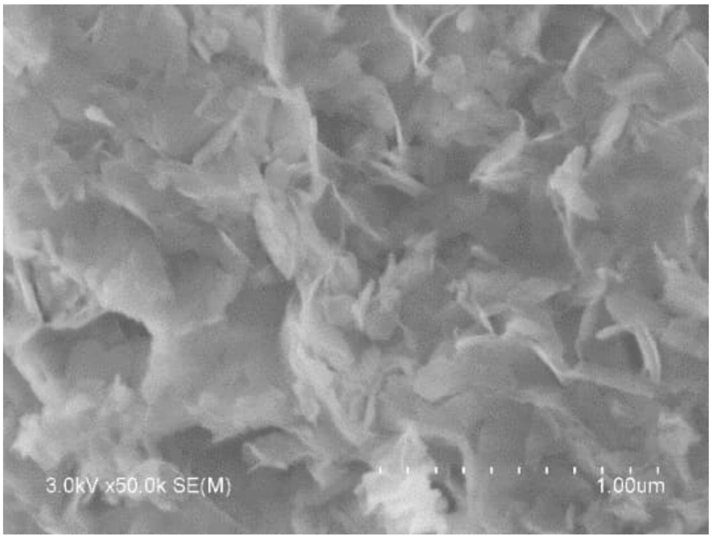


Fig. 2

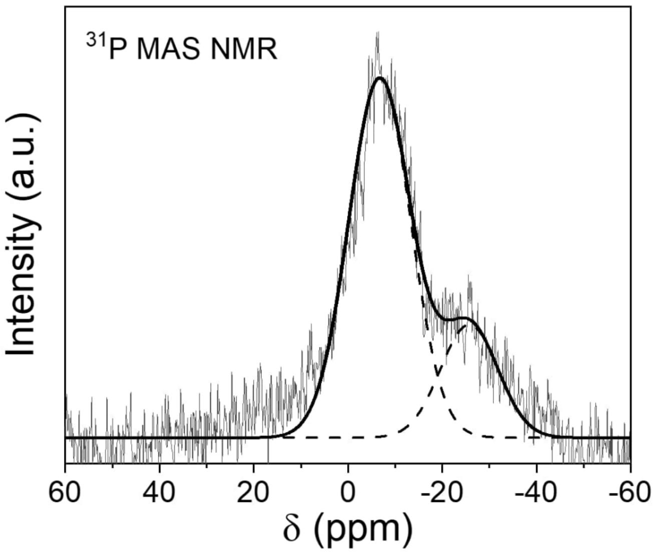


Fig. 3

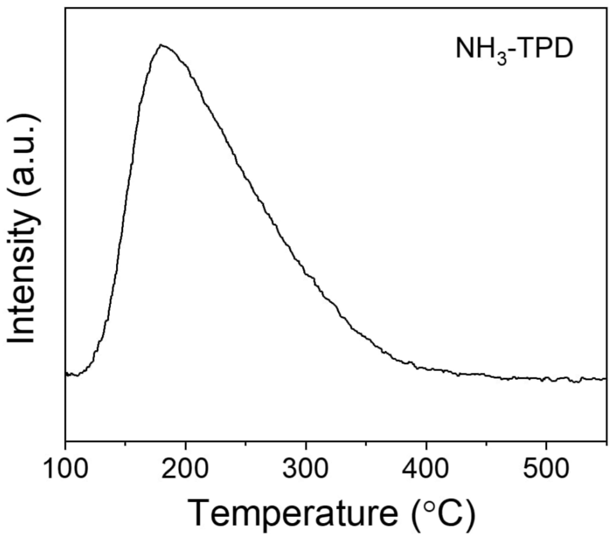


Fig. 4

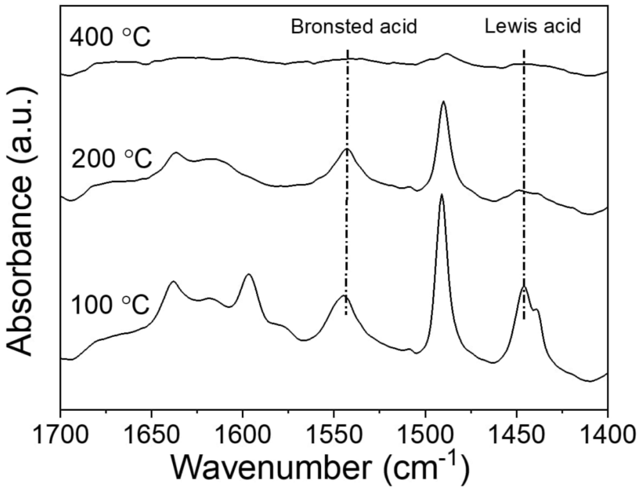
400 °C
Bronsted acid
Lewis acid
200 °C
100 °C
Absorbance (a.u.)
1700
1650
1600
1550
1500
1450
1400
Wavenumber (cm-1)

Fig. 5

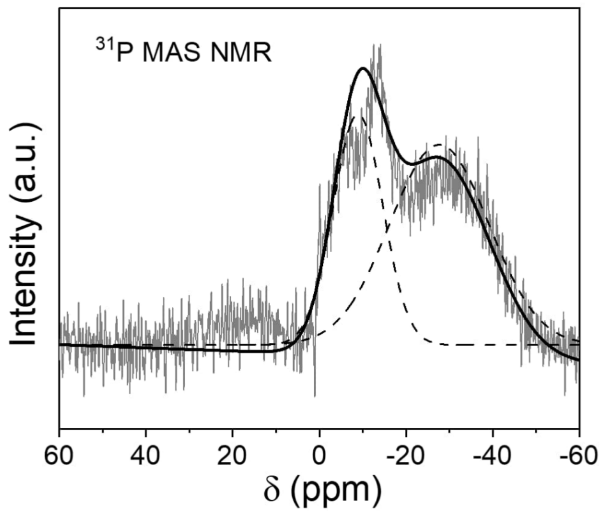
31P MAS NMR
Intensity (a.u.)
60
40
20
0
-20
-40
-60
δ (ppm)

Fig. 6

**INTERNATIONAL SEARCH REPORT**

International application No.
**PCT/CN2023/142795**

**A. CLASSIFICATION OF SUBJECT MATTER**

C01B39/10(2006.01)i; C07C2/86(2006.01)i

According to International Patent Classification (IPC) or to both national classification and IPC

**B. FIELDS SEARCHED**

Minimum documentation searched (classification system followed by classification symbols)

IPC:C01B,C07C

Documentation searched other than minimum documentation to the extent that such documents are included in the fields searched

Electronic data base consulted during the international search (name of data base and, where practicable, search terms used)

CNABS, CNTXT, VEN, WOTXT, USTXT, EPTXT, CNKI, Web of science: 中国石油化工股份有限公司, 中石化(上海)石油化工研究院有限公司, 杨为民, 冯心强, 李相呈, 王振东, 刘闯, 徐睿, 韩笑, 分子筛, MCM, ITQ, SCM, MWW, 磷修饰, P修饰, P改性, 磷改性, P掺杂, 磷掺杂, H型, 水蒸气, molecular sieve, phosphor, modification, doped, H-type, steam

**C. DOCUMENTS CONSIDERED TO BE RELEVANT**

| Category* | Citation of document, with indication, where appropriate, of the relevant passages | Relevant to claim No. |
|---|---|---|
| Y | CN 115477310 A (CHINA PETROLEUM & CHEMICAL CORPORATION et al.) 16 December 2022 (2022-12-16)<br>description, paragraphs 5-25 | 1-10 |
| Y | CN 115385354 A (CHINA PETROLEUM & CHEMICAL CORPORATION et al.) 25 November 2022 (2022-11-25)<br>description, paragraphs 8-39 | 1-10 |
| Y | CN 116003200 A (CHINA PETROLEUM & CHEMICAL CORPORATION et al.) 25 April 2023 (2023-04-25)<br>description, paragraphs 4-54 | 10 |
| A | CN 113880102 A (FUZHOU UNIVERSITY) 04 January 2022 (2022-01-04)<br>entire document | 1-10 |
| A | CN 114715910 A (CHINA PETROLEUM & CHEMICAL CORPORATION et al.) 08 July 2022 (2022-07-08)<br>entire document | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 March 2024** | **23 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2023/142795

C. DOCUMENTS CONSIDERED TO BE RELEVANT

| Category* | Citation of document, with indication, where appropriate, of the relevant passages | Relevant to claim No. |
|---|---|---|
| A | CN 115109608 A (DALIAN INSTITUTE OF CHEMICAL PHYSICS, CHINESE ACADEMY OF SCIENCES) 27 September 2022 (2022-09-27)<br>entire document | 1-10 |
| A | WO 2022184759 A1 (EXXONMOBIL CHEMICAL PATENTS INC. et al.) 09 September 2022 (2022-09-09)<br>entire document | 1-10 |

Form PCT/ISA/210 (second sheet) (July 2022)

**INTERNATIONAL SEARCH REPORT**
**Information on patent family members**

International application No.
**PCT/CN2023/142795**

| Patent document cited in search report | Publication date (day/month/year) | Patent family member(s) | Publication date (day/month/year) |
|---|---|---|---|
| CN 115477310 A | 16 December 2022 | CN 115477310 B | 28 November 2023 |
| CN 115385354 A | 25 November 2022 | CN 115385354 B | 09 January 2024 |
| CN 116003200 A | 25 April 2023 | CN 115959681 A | 14 April 2023 |
| | | TW 202315841 A | 16 April 2023 |
| | | WO 2023061256 A1 | 20 April 2023 |
| CN 113880102 A | 04 January 2022 | None | |
| CN 114715910 A | 08 July 2022 | CN 114715910 B | 05 May 2023 |
| CN 115109608 A | 27 September 2022 | CN 115109608 B | 24 October 2023 |
| WO 2022184759 A1 | 09 September 2022 | EP 4301700 A1 | 10 January 2024 |
| | | CA 3207884 A1 | 09 September 2022 |
| | | JP 2024509153 A | 29 February 2024 |
| | | CN 117043108 A | 10 November 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 113831238 A
- CN 113831308 A
- CN 103814005 A
- CN 102482177 B
- WO 2014043468 A1
- CN 109569677 B
- CN 107626341 A
- CN 115385771 A
- CN 102233274 A
- US 20140194276 A1
- CN 109678172 A
- CN 106517232 B
- WO 1997017290 A1
- CN 104511271 B